# FASCICULE DE BREVET EUROPEEN

(11) **EP 3 179 859 B1**
(45) Date de publication et mention de la délivrance du brevet: **31.10.2018**
(21) Numéro de dépôt: 15750050.5
(22) Date de dépôt: 12.08.2015
(51) Int. Cl.: A01N 63/02, A01K 67/033, A01N 25/04

(54) **COMPOSITION COMPRENANT DES OEUFS D'ARTHROPODES**
ARTHTROPODENEIER-ENTHALTENDE ZUSAMMENSETZUNG
COMPOSITION COMPRISING ARTHROPOD EGGS

(30) Priorité: 12.08.2014 FR 1457771
(43) Date de publication de la demande: 21.06.2017
(73) Titulaire: AgroSolutions, 75016 Paris (FR)
(72) Inventeur: FERRERO, Maxime, F-06620 Bar sur Loup (FR); MAIGNET, Pascal, F-06460 Saint Vallier de Thiey (FR); BONHOMME, Antoine, F-92500 Rueil-Malmaison (FR)
(74) Mandataire: Regimbeau
(86) Numéro de dépôt international: PCT/EP2015/068588
(87) Numéro de publication internationale: WO 2016/023957

(56) Documents cités:
- WO-A1-2011/089105
- WO-A1-2013/048251
- WO-A1-2013/190142
- US-A- 5 718 377
- MICHAEL P SEAGRAVES ET AL: "Sugar feeding by coccinellids under field conditions: the effects of sugar sprays in soybean", BIOCONTROL, KLUWER ACADEMIC PUBLISHERS, DO, vol. 56, no. 3, 5 décembre 2010 (2010-12-05), pages 305-314, XP019907639, ISSN: 1573-8248, DOI: 10.1007/S10526-010-9337-3

## Description

La présente invention appartient au domaine de la lutte biologique, et en particulier des produits et méthodes de lutte biologique mettant en oeuvre des agents de contrôle biologiques qui sont des arthropodes.

L'utilisation d'agents de contrôle biologique constitue une méthode de lutte contre des ravageurs, des agents pathogènes ou des plantes adventices au moyen d'organismes naturels antagonistes de ceux-ci, tels que des phytophages (dans le cas d'une plante adventice), des parasitoïdes (dans le cas des arthropodes...), des prédateurs (dans le cas des nématodes, arthropodes, vertébrés, mollusques, chauves-souris...) ou des agents pathogènes (dans le cas des virus, bactéries, champignons...).

L'utilisation de ces agents de contrôle biologique permet également une réduction de l'utilisation de pesticides.

Habituellement, les agents de contrôle biologique sont introduits dans les cultures à protéger sous forme de compositions présentées dans des sachets comprenant les prédateurs accompagnés des aliments leur permettant de se nourrir pendant une certaine période, ou alors en vrac, sous forme de bouteilles qui permettent de déposer des quantités variables de prédateurs sur les plantes ou au sol. Les prédateurs colonisent ensuite les cultures, où ils se nourrissent alors des proies visées.

Toutefois, dans la majorité des cas, les populations d'agents de contrôle biologique périclitent après un certain temps passé sur les plantes. Il s'avère par conséquent nécessaire de réintroduire les prédateurs à intervalles réguliers, de manière à obtenir une protection continue.

Des préparations alimentaires permettant de nourrir les agents de contrôle biologique ont été mises au point, afin de tenter de contourner ce problème. Par exemple, la demande WO2013/48251 décrit des capsules de gélatine comprenant des compositions à base de sucre, permettant de nourrir par exemple des insectes zoophytophages.

Toutefois, ces gélules ont tendance à s'accumuler au pied des cultures, et sont par conséquent difficiles à appliquer à proximité des agents de culture biologique, lesquels sont disséminés sur toute la plante. En outre, les compositions à base de sucre sont des aliments qui ne sont pas appropriés à tous les prédateurs usuellement utilisés dans le domaine de la culture biologique.

Il existe donc toujours un besoin pour des moyens améliorés d'augmenter la durée de vie des populations d'agents de contrôle biologique *in situ,* donc de permettre une installation durable de ces agents en serre ou en champs. US5718377 décrit la pulvérisation dans les champs d'oeufs d'insectes utiles: il ne sont pas censés servir d'aliment, mais la technique employée montre que de tels oeufs peuvent être pulvérisés sans dommage. En outre, US5718377 envisage l'intérêt d'accroître la pégosité de la composition pulvérisée, p.ex. au moyen de farine de maïs prégélatinisée.

Les inventeurs ont découvert que des compositions à base d'oeufs d'arthropodes, en particulier d'acariens, d'insectes, ou de crustacés, et de gommes végétales sont particulièrement efficaces pour améliorer la durée de vie des populations de prédateurs habituellement utilisés dans le domaine de la lutte biologique.

Ainsi, les inventeurs ont mis au point des compositions qui sont suffisamment fluides pour être pulvérisées et suffisamment visqueuses après la pulvérisation pour permettre d'en enduire les feuilles et tiges des plantes à traiter, tout en étant particulièrement appréciées des prédateurs. En effet, la fécondité et la longévité des populations de prédateurs nourries avec de telles compositions sont très supérieures à celles des populations non traitées.

Les inventeurs ont de plus montré que des résultats analogues peuvent être obtenus en préparant la composition de l'invention avec des composés possédant des propriétés similaires aux gommes végétales, mais n'entrant pas dans cette catégorie. Ces composés, choisis parmi les épaississants, les gélifiants, les stabilisateurs et les émulsifiants, permettent d'obtenir des compositions visqueuses, lesquelles sont facilement répandues sur l'ensemble de la plante.

La présente invention a pour premier objet une composition aqueuse pulvérisable, destinée à nourrir des agents de contrôle biologique, comprenant des oeufs d'au moins une espèce d'arthropodes et un ou plusieurs épaississants et/ou gélifiants.

L'invention a pour deuxième objet un procédé de préparation d'une composition selon l'invention.

L'invention a pour troisième objet l'utilisation d'une composition selon l'invention, en tant que source de nutriment pour des agents de contrôle biologique qui sont des arthropodes. L'invention a pour autre objet l'utilisation d'une composition selon l'invention, pour améliorer l'efficacité des procédés de contrôle biologique.

L'invention a pour autre objet une méthode de contrôle biologique comprenant une étape d'application sur les plantes d'une composition selon l'invention.

### Figures :

FIGURE 1. Pourcentages d'oeufs viables restants sur des plants de poivrons pulvérisés avec des oeufs *d'Aleuroglyphus ovatus* mélangés soit à 1% en poids de gomme d'acacia par volume d'eau soit à de l'eau.
FIGURE 2. Comparaison du nombre moyen d'*A*. *swirskii* par plant de concombre avec ou sans pulvérisation d'oeufs d'*A*. *ovatus* mélangés à 1% en poids de gomme d'acacia par volume d'eau comme nourriture alternative.
FIGURE 3. Comparaison du nombre moyen d'*A*. *swirskii* par plant de poivron avec ou sans pulvérisation d'oeufs d'*A*. *ovatus* mélangés à 1% en poids de gomme d'acacia par volume d'eau comme nourriture alternative.

### Définitions :

On entend par «composition aqueuse» une composition comprenant 10 à 90%, de préférence 20 à 80%, de manière particulièrement préférée 30 à 70%, de manière encore préférée 60 à 70% d'eau.

Par « pulvérisable », on entend projetable par des pulvérisateurs grande culture généralement utilisés en champs, ou par des pulvérisateurs manuels utilisés dans les jardins ou cultures spécialisées.

Par l'expression « oeuf », on entend au sens de la présente invention, les oeufs récoltés et non encore éclos. Par exemple, les oeufs peuvent être utilisés frais ou après avoir été traités par tout mode de conservation permettant de maintenir toutes les propriétés nutritives de l'oeuf. Préférentiellement, les oeufs sont traités par tout mode de conservation permettant de maintenir l'oeuf dans son stade de développement.

Par exemple, afin de prévenir leur développement ultérieur, les oeufs peuvent être congelés.

Alternativement, afin de prévenir leur éclosion, les oeufs peuvent être spécifiquement traités de manière à induire un état dit « sublétal ». Selon la présente invention, par « oeufs sublétaux » il est fait référence à des oeufs qui ne peuvent éclore mais conservent toutefois une activité métabolique. Un procédé d'obtention de tels oeufs sublétaux est décrit notamment dans WO 2013/190142.

Selon l'invention, le terme « viscosité dynamique» se réfère à la viscosité mesurée par les outils usuels (viscosimètre), à une température d'environ 20°C (notamment ±1°C). Une telle viscosité peut être mesurée notamment à l'aide d'un viscosimètre de type Brookfield à une vitesse de rotation de 5 tr/min.

### Description détaillée

La présente invention a pour premier objet une composition aqueuse pulvérisable, destinée à nourrir des agents de contrôle biologique, comprenant des oeufs d'au moins une espèce d'arthropodes et un ou plusieurs épaississants et/ou gélifiants.

Selon l'invention, les oeufs peuvent être entiers, ou être fractionnés. Les oeufs peuvent être traités de manière à extraire des nutriments particuliers.

Les oeufs de l'invention constituent au sens propre une population d'arthropodes, il est donc aussi possible de définir la composition de l'invention comme comprenant :
- une population d'arthropodes, laquelle population consiste en des oeufs d'au moins une espèce d'arthropodes,
- un ou plusieurs épaississants et/ou gélifiants
- de l'eau.

Le terme « population d'arthropodes » selon l'invention signifie toute population comprenant au moins deux individus appartenant à au moins une des espèces d'arthropodes, de préférence sélectionnées parmi les insectes, les acariens, ou les crustacés.

Selon l'invention, la population d'arthropodes consiste uniquement en individus encore au stade d'oeuf. Ainsi, au sens de l'invention, la population d'arthropodes ne comprend pas d'arthropodes au stade de larve, de nymphe, ni d'individu adulte.

Selon un mode de réalisation préféré de l'invention, la composition ne comprend, en tant qu'arthropodes, que des oeufs, et ne comprend pas d'arthropodes au stade de larve, de nymphe, ni d'individu adulte.

Ainsi, selon les modes de réalisation envisagés par l'homme du métier, lesdits oeufs d'arthropodes appartiennent à une ou plusieurs espèce(s) d'acarien, ou une ou plusieurs espèce(s) d'insecte, ou une ou plusieurs espèce(s) de crustacé. L'invention vise de plus les modes de réalisation dans lesquels lesdits oeufs d'arthropodes appartiennent à au moins une espèce d'acarien et au moins une espèce d'insecte, ou au moins une espèce d'acarien et au moins une espèce de crustacé, ou au moins une espèce d'insecte et au moins une espèce de crustacé.

L'invention vise aussi les modes de réalisation dans lesquels la population d'arthropodes comprend au moins une espèce d'acarien, au moins une espèce d'insecte, au moins une espèce de crustacé.

L'invention vise en outre les modes de réalisation dans lesquels la population d'arthropodes consiste en des oeufs provenant de la même classe ou du même sous-embranchement. Par exemple, l'invention vise les modes de réalisation dans lesquels la population d'arthropodes consiste en des oeufs d'acariens, ou en des oeufs d'insectes, ou en des oeufs de crustacés.

L'invention vise en outre les modes de réalisation dans lesquels la population d'arthropodes consiste en une unique espèce d'arthropodes. Par exemple, l'invention vise les modes de réalisation dans lesquels la population d'arthropodes consiste en des oeufs d'une espèce d'arthropode choisie parmi la liste consistant en les espèces d'acariens, les espèces d'insectes, et les espèces de crustacés.

Selon un mode de réalisation, les acariens sont choisis parmi la famille des Tetranychidae, de préférence du genre *Tetranychus*.

Selon un mode de réalisation, les arthropodes sont choisis parmi les astigmates, par exemple de la famille des *Pyroglyphidae*, de la famille des *Acaridae,* de la famille des *Carpoglyphidae*, ou de la famille des *Glyciphagidae*.

Selon un mode de réalisation avantageux, les astigmates sont choisis parmi la famille des *Acaridae,* par exemple le genre Acarus, à titre d'illustration *Acarus siro*, *Acarus farris, Acarus immobilis* ou *Acarus chaetoxysilos*; ou selon un autre exemple au moins le genre *Tyrophagus,* comme *Tyrophagus longior*, *Tyrophagus similis* ou *Tyrophagus putrescentiae*; ou encore le genre *Aleuroglyphus*, tel *Aleuroglyphus ovatus*; ou bien le genre *Lardoglyphus*, comme par exemple *Lardoglyphus konoi*; le genre *Caloglyphus*, tel *Caloglyphus mycophagus* ou *Caloglyphus berlesei*; le genre *Suidasia*, comme par exemple *Suidasia nesbitti*; le genre *Thyreophagus,* tel *Thyreophagus entomophagus;* la famille Carpoglyphidae, comme par exemple le genre *Carpoglyphus*, par exemple *Carpoglyphus lactis*, *Carpoglyphus munroi*; la famille Glycyphagidae, comme par exemple le genre *Glycyphagus*, tel *Glycyphagus domesticus*; le genre *Lepidoglyphus*, tel *Lepidoglyphus destructor*; le genre *Blomia*, comme par exemple *Blomia freemani*; la famille Chortoglyphydae, le genre *Chortoglyphus*, par exemple *Chortoglyphus arcuatus*.

Avantageusement, les oeufs d'astigmates sont choisis parmi au moins une espèce du genre *Aleuroglyphus*. De façon particulièrement avantageuse, les oeufs d'astigmates sont de l'espèce *Aleuroglyphus ovatus*.

Selon un mode de réalisation, les arthropodes sont choisis parmi les insectes, en particulier les lépidoptères et les diptères par exemple la mouche domestique (Mosca domestica) et les Tephritidae (genres Ceratitis, Dacus, Bactrocera).

Selon un mode de réalisation de l'invention, les lépidoptères sont choisis par exemple dans la famille des *Pyralidae*, par exemple *Ephestia kuhniella*, *Ephestia elutella*, *Ephestia cautella*, *Corcyra cephalonica* ou *Plodia interpunctella*, ou par exemple de la sous-famille des *Gelechiidae,* par exemple *Sitotroga cerealella*. Selon un mode de réalisation particulièrement avantageux, les lépidoptères sont des oeufs d'*Ephestia kuhniella*.

Selon un mode de réalisation, les arthropodes sont choisis parmi le sous embranchement des *Crustacea*, par exemple de la classe des *Branchiopoda*, de la classe des *Cephalocarida*, de la classe des *Malacostraca*, de la classe des *Maxillopoda*, de la classe des *Ostracoda*, de la classe des *Remipedia*.

La composition de l'invention comprend un ou plusieurs épaississants et/ou gélifiants. Avantageusement, les épaississants et gélifiants utilisés dans la composition selon l'invention sont présents dans la composition à raison de 0,01 à 5%, de préférence de 0,05 à 4 %, de préférence de 0,10 à 3%, de manière particulièrement préférée de 0,2 à 0,5% en poids par rapport au volume de la composition.

A titre illustratif, la composition selon l'invention pourra comprendre 0,01 à 5 % p/v de maltodextrines, de préférence de 0,02 à 4 % p/v, de manière particulièrement préférée de 0,03 à 3,5 % p/v.

A titre illustratif, la composition selon l'invention pourra comprendre 0,01 à 5 % p/v de glucomannanes, de préférence de 0,05 à 4 % p/v, de manière particulièrement préférée de 0,1 à 3 % p/v.

A titre illustratif, la composition selon l'invention pourra comprendre 0,01 à 5 % p/v de production exogène de *Sclerotium rolfsii*, de préférence de 0,1 à 3 % p/v, de manière particulièrement préférée de 0,25 à 2,50 % p/v.

A titre illustratif, la composition selon l'invention pourra comprendre 0,01 à 5 % p/v de gomme d'acacia, de préférence de 0,02 à 4 % p/v, de manière particulièrement préférée de 0,05 à 3 % p/v.

Les épaississants et/ou gélifiants sont présents dans la composition selon l'invention en une quantité apportant à la composition une viscosité dynamique à gradient de vitesse nulle comprise entre 0,005 et 100 Pa.s, en particulier entre 0,02 et 50 Pa.s, de préférence entre 0,03 et 20 Pa.s, de manière encore préférée entre 0,04 et 10 Pa.s, de manière particulièrement préférée entre 0,05 et 5 Pa.s. En particulier, les épaississants et/ou gélifiants sont présents dans la composition selon l'invention en une quantité apportant à la composition une viscosité dynamique à gradient de vitesse nulle comprise entre 0,05 et 100 Pa.s, de préférence entre 0,1 et 50 Pa.s, de manière encore préférée entre 0,2 et 10 Pa.s, de manière particulièrement préférée entre 0,5 et 1 Pa.s.

La composition selon l'invention présente après sa préparation une viscosité adaptée à sa pulvérisation puis sa viscosité augmente après sa pulvérisation pour adhérer à ce sur quoi elle a été pulvérisée, par exemple aux feuilles et tiges des plantes.

Les épaississants et gélifiants utilisés dans la composition selon l'invention préférentiellement ne sont pas des épaississants et gélifiants devant être utilisés à chaud afin d'éviter tout risque de détérioration des oeufs contenus dans la composition.

Par « épaississants et gélifiants devant être utilisés à chaud », on entend, au sens de la présente invention, des épaississants et gélifiants dont l'utilisation implique une étape à chaud, c'est-à-dire au-dessus de 40°C, notamment au-dessus de 35°C, lors de la préparation des compositions selon l'invention, et ce en présence des oeufs d'au moins une espèce d'arthropodes. Un tel épaississant ou gélifiant est notamment l'agar-agar.

Les épaississants et gélifiants utilisés dans la composition selon l'invention sont préférentiellement d'origine végétale, de manière particulièrement préférée ce sont des gommes végétales.

Par « épaississants et gélifiants d'origine végétale », on entend :
- par exemple la gomme arabique, la gomme adragante, la gomme karaya,
- la gomme de guar, la gomme de caroube, la gomme tara, la gomme d'avoine,
- des glucomannanes comme le glucomannane de konjac, l'hydroxypropylcellulose, le sel sodique de la carboxyméthyl cellulose,
- les produits de la production exogène de *Sclerotium rolfsii*,
- des maltodextrines comme par exemple les maltodextrines de maïs, les maltodextrines de tapioca, les maltodextrines de pomme de terre,
- la gomme d'acacia, la gomme xanthane, la gomme gellane, la gomme ghatti, la gomme de konjac, la gomme de casse.

Par « gomme végétale », on entend au sens de l'invention un composé glucidique adhésif fabriqué à l'intérieur des tissus végétaux par des cellules spécialisées. La gomme végétale est une matière visqueuse exsudée par certaines plantes, et qui peut par conséquent facilement être récoltée. Les gommes végétales sont bien connues pour leurs propriétés épaississantes, stabilisatrices, gélifiantes, et/ou émulsifiantes.

Les gommes végétales au sens de l'invention sont préférentiellement choisies parmi la liste consistant en la gomme d'acacia, la gomme de caroube, la gomme d'avoine, la gomme de guar, la gomme adragante, la gomme arabique, la gomme xanthane, la gomme karaya, la gomme tara, la gomme gellane, la gomme ghatti, la gomme de konjac, la gomme de casse.

Les épaississants et gélifiants utilisés dans la composition selon l'invention peuvent également être choisis parmi les maltodextrines, en particulier les maltodextrines de maïs, les maltodextrines de tapioca, les maltodextrines de pomme de terre, le sel sodique de la carboxyméthyl cellulose, le glucomannane, une poudre de graines ou de téguments de psyllium, les produits de la production exogène de *Sclerotium rolfsii*, le xanthane, l'hydroxypropylcellulose.

La composition de l'invention peut en outre comprendre d'autres composés d'intérêts.

Par exemple, la composition de l'invention peut comprendre des sources additionnelles de nutriments, en plus des oeufs d'arthropodes, tels que par exemple du pollen ou des vitamines.

La composition de l'invention peut aussi comprendre des antioxydants ou des conservateurs.

A titre d'exemple, sans que cela limite en rien l'objet de l'invention, les antioxydants peuvent être choisis parmi les antioxydants utilisés dans l'industrie agroalimentaire, tels que par exemple parmi la liste consistant en l'acide ascorbique ou vitamine C (E300), les ascorbates de sodium (E301), les ascorbates de calcium (E302), l'acide diacétyl 5-6-1-ascorbique (E303), l'acide palmityl 6-1-ascorbique (E304), l'acide citrique (E330), les citrates de sodium (E331), les citrates de potassium (E332), les citrates de calcium (E333), l'acide tartrique (E334), les tartrates de sodium (E335), les tartrates potassium (E336) et les tartrates desodium et de potassium (E337), le butylhydroxyanisol (E320), le butylhydroxytoluol (E321), les gallates d'octyle (E311), les gallates dodécyle (E312), les lactates de sodium (E325), les lactates de potassium (E326), les lactates de calcium (E327), les lécithines (E322), les tocophérols naturels (E306), l'alpha-tocophérol de synthèse (E307), le gamma-tocophérol de synthèse (E308), le delta-tocophérol de synthèse (E309), et l'ensemble des tocophérols constituant la vitamine E.

Selon l'invention, les conservateurs peuvent être choisis parmi les conservateurs alimentaires, en particulier choisis dans la liste consistant en les nitrates et nitrites (E249 - E252), les sulfites (E221 - E228), l'anhydride sulfureux (E220), le peroxyde d'hydrogène, l'acide sorbique (E200), l'acide benzoïque (E210), l'acide fumarique (E297), l'erythorbate de sodium (E316), les parabènes (E214 - E219), le dicarbonate de diméthyle (E242).

Un autre objet de l'invention concerne un procédé de préparation d'une composition selon l'invention.

Ce procédé comprend les étapes successives de :
- dissolution de ou des épaississants et/ou gélifiants dans une base aqueuse, de préférence dans l'eau,
- ajout des oeufs d'arthropodes à ce mélange.

La composition de l'invention est particulièrement intéressante pour nourrir les prédateurs usuellement utilisés dans le domaine de la lutte biologique, que ce soit dans le cadre de l'élevage de ces prédateurs, ou bien ultérieurement, en serre ou en champs, après que ceux-ci aient été répandus sur les cultures à traiter.

Ainsi, un autre objet de l'invention est l'utilisation de la composition de l'invention en tant que source de nutriment pour des agents de contrôle biologique.

Les inventeurs ont établi que la composition de l'invention permet d'augmenter la durée de vie et la reproduction des agents de contrôle biologique, dans le cadre de leur élevage, mais aussi ultérieurement, en serre ou en champs, après que ceux-ci ont été répandus sur les cultures à traiter. L'utilisation de la composition de l'invention permet par conséquent de diminuer la fréquence d'épandage des agents de contrôles biologiques, voire de la limiter à un seul lâcher en début de culture, et de manière générale, d'améliorer l'efficacité des procédés de contrôle biologique.

Ainsi, un autre objet de l'invention est l'utilisation de la composition de l'invention pour améliorer l'efficacité des procédés de contrôle biologique.

Les inventeurs ont en particulier déterminé que la composition de l'invention est particulièrement appréciable pour nourrir les agents de contrôle biologique qui sont des arthropodes particuliers. En effet, il a été observé une meilleure croissance de ce type de prédateurs, lorsque ceux-ci sont nourris avec la composition de l'invention.

Selon un mode de réalisation, les agents de contrôle biologique sont choisis parmi la classe des *Arachnida*, préférentiellement parmi les acariens (ordre des *Acari*), ou parmi la classe des insectes.

Selon un mode de réalisation, les acariens peuvent être choisis par exemple parmi les familles des phytoséiides *(Phytoseiidae)*, des *Macrochelidae*, des *Laelapidae* ou encore des Cheyletidae.

Avantageusement, les phytoséiides peuvent être choisis parmi la sous-famille des Amblyseiinae, par exemple *Amblyseius swirskii*, *Amblyseius largoensis*, *Amblyseius andersoni*; ou parmi le genre *Neoseiulus*, e.g. *Neoseiulus womersleyi*, *Neoseiulus californicus*, *Neoseiulus cucumeris*, *Neoseiulus fallacis*, *Neoseiulus longispinosus*; ou parmi le genre *Iphiseius*, e.g. *Iphiseius degenerans*; ou parmi le genre *Amblydromalus*, par exemple *Amblydromalus lailae*, *Amblydromalus limonicus* ou *Amblydromalus manihoti*, ou parmi le genre *Phytoseiulus*, par exemple *Phytoseiulus persimilis*, *Phytoseiulus macropilis* ou *Phytoseiulus longipes* ; ou parmi la sous-famille des Typhlodrominae, en particulier le genre *Typhlodromips*, par exemple *Typhlodromips montdorensis*; ou parmi le genre *Euseius*, par exemple *Euseius ovalis*, *Euseius scutalis*, *Euseius finlandicus*, *Euseius gallicae*, *Euseius stipulatus*, *Euseius tularensis*, *Euseius hibisci*.

Avantageusement, les acariens de la famille des Macrochelidae peuvent être choisis parmi les *Macrocheles*, *par exemple Macrocheles robustulus*.

Avantageusement, les acariens de la famille des Laelapidae peuvent être choisis parmi les *Hypoaspis*, par exemple *Gaeolaelaps aculeifer ou Stratiolaelaps scimitus*.

Avantageusement, les acariens de la famille des Laelapidae peuvent être choisis parmi les *Cheletus*, par exemple *Cheletus eruditus*.

Préférentiellement, les acariens sont choisis parmi *Amblyseius swirskii, Neoseiulus cucumeris*, *Amblyseius largoensis*, *Neoseiulus californicus*, *Neoseiulus fallacis*, *Neoseiulus longispinosus*, *Amblydromalus lailae*, *Typhlodromips montdorensis*, *Euseius ovalis*, *Euseius scutalis*, *Euseius hibisci*. Selon un mode de réalisation particulièrement avantageux de l'invention, les acariens sont de l'espèce *Amblyseius swirskii*.

L'invention a aussi pour objet une méthode de contrôle biologique comprenant une étape d'application sur les plantes d'une composition selon l'invention.

La composition amène une source de nutriment aux agents de contrôle biologique déjà présents dans les cultures.

Ainsi, la composition de l'invention est appliquée sur les plantes en vue de nourrir un agent de contrôle biologique qui y a été précédemment introduit ou qui est concomitamment introduit.

Ainsi, selon ce mode de réalisation, la méthode de contrôle biologique de l'invention, comprend les étapes successives ou concomitantes de :
a) introduction d'agents de contrôle biologique dans la culture;
b) application d'une composition selon l'invention sur les plantes de ladite culture.

Avantageusement, la composition selon l'invention est appliquée de nouveau, au maximum deux fois par mois, de préférence une fois par mois, après sa première application. Ainsi, les agents de contrôle biologique introduits une fois au début de la méthode selon l'invention sont maintenus par une source de nutriment constante.

L'application de la composition selon l'invention pourra être réalisée sur tout type de culture, en particulier sur les cultures en serres et sur les cultures en champs. En effet, la composition de l'invention permet de traiter les plantes quelles que soient les conditions de culture.

L'application pourra avantageusement être réalisée à l'aide d'un pulvérisateur, en particulier à l'aide d'un pulvérisateur grande culture, ou par des pulvérisateurs manuels utilisés dans les jardins ou cultures spécialisées.

Après son application, la composition pulvérisable selon l'invention devient visqueuse.

Les exemples suivants illustrent l'invention sans en limiter la portée.

### Exemples

### Exemple 1 : Les oeufs d'astigmates mélangés à une préparation pulvérisable résistent mieux à la dessiccation que les oeufs d'astigmates mélangés à de l'eau

### Protocole

On prépare une solution de gomme d'acacia dans de l'eau à raison de 1% en poids de gomme d'acacia /volume d'eau.

Des oeufs d'acariens de l'espèce *Aleuroglyphus ovatus*, conservés au préalable à +3°C pendant 6 semaines, ont été mélangés à une préparation de 1% en poids de gomme d'acacia par volume d'eau.

La solution obtenue a été pulvérisée à l'aide d'un petit pulvérisateur (200 ml) sur la face inférieure des feuilles de 4 plants de poivrons de 25 cm disposés dans une serre. A 7 et 12 jours après pulvérisation, une feuille de chaque plante a été prélevée, et le nombre d'oeufs affaissés et turgescents ont été comptés.

Un thermohygromètre a également été disposé au sein du dispositif pour évaluer les températures et hygrométries extrêmes auxquelles les oeufs auront été soumis.

### Résultats

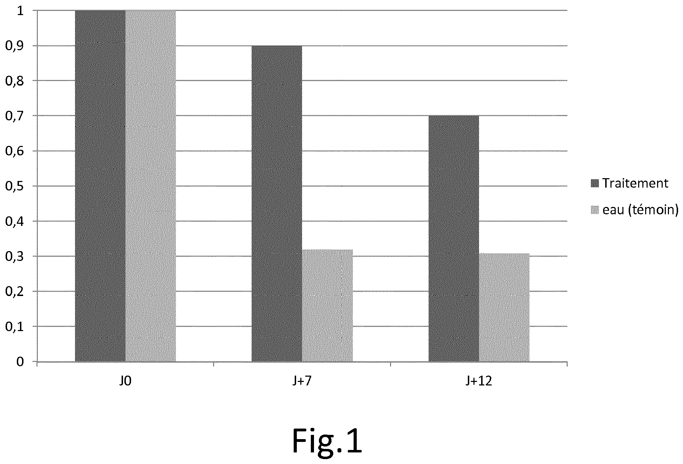

Les pourcentages d'oeufs viables restants sur les feuilles, issus des moyennes des comptages d'oeufs réalisés sont présentés en FIGURE 1 (témoin = eau).

Les minimum et maximum des températures et humidités relatives observées dans le dispositif sur les douze jours de l'expérimentation ont varié respectivement de 9 à 36 °C, et de 13 à 93 % d'humidité relative (HR).

### Conclusion

La FIGURE 1 montre que des oeufs mélangés à une préparation pulvérisable qui devient visqueuse après application s'affaissent moins vite que des oeufs mélangés à l'eau, et qu'ils résistent donc mieux à la dessiccation, dans des conditions de température et d'humidité très variables.

Après 12 jours d'exposition, il reste en effet toujours 70 % d'oeufs viables, alors que seuls 30 % des oeufs mélangés à l'eau sont encore turgescents.

### EXEMPLE 2: Appétence des oeufs d'arthropodes mélangés à une préparation fluide pulvérisable pour les acariens prédateurs

### Protocole

36 plants de concombres et 36 plants de poivrons ont été plantés dans une serre, en hors sol sur des pains de substrat, avec une irrigation contrôlée. 3 plants ont été plantés par pain.

On prépare une solution de gomme d'acacia dans de l'eau à raison de 1% en poids de gomme d'acacia /volume d'eau.

A J0, un pain sur deux (3 plantes oui, 3 plantes non) a été pulvérisé avec une solution selon l'exemple 1 contenant des oeufs d'*A*. *ovatus* et 1% en poids de gomme d'acacia /volume d'eau. Cinq femelles d'*A*. *swirskii* ont ensuite été déposées sur chaque plante (pulvérisée ou non). Par la suite, les plantes ayant été précédemment pulvérisées ont été pulvérisées à nouveau à J+15 et J+30.

Un comptage du nombre d'*A*. *swirskii* par feuille a été effectué deux fois par semaine, sur 5 feuilles (poivron) ou 3 feuilles (concombre) par plante, pendant 35 jours.

### Résultats

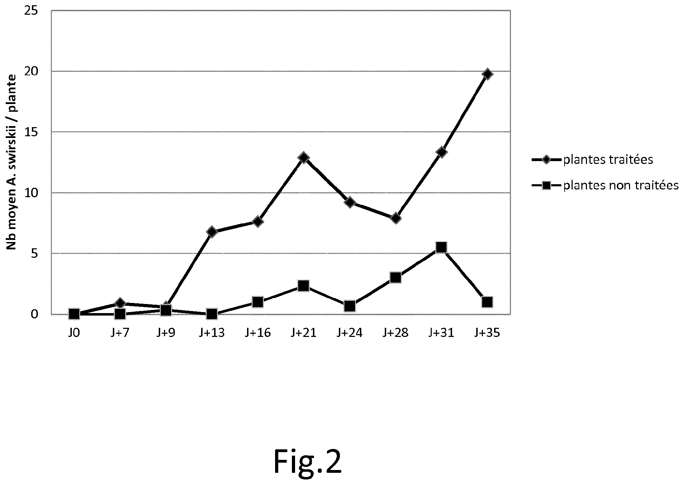

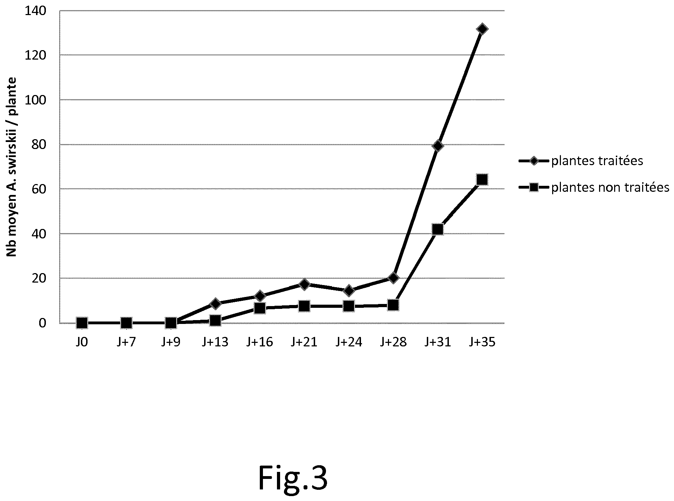

Le nombre d'*A*. *swirskii* par plant de concombre ou de poivron issus des comptages effectués sont présentés respectivement sur les FIGURE 2 et 3.

### Conclusions

Les résultats montrent que *A*. *swirskii* est capable de se nourrir et de se reproduire dans des cultures en serre avec une composition fluide mais visqueuse contenant des oeufs d'A. ovatus , comme l'attestent les FIGURES 2 et 3, qui montrent une augmentation des populations d'*A*. *swirskii* sur les plantes traitées, quelle que soit la culture.

Sur une culture de poivron (FIGURE 3), en l'absence de nourriture alternative, *A*. *swirskii* est capable de se maintenir en se nourrissant du pollen de la plante, jusqu'à des niveaux atteignant plus de 60 individus / plante. En présence d'oeufs d'*A*. *ovatus* mélangés à la solution de gomme d'acacia, non seulement cette installation est plus rapide (meilleur effet préventif), mais le nombre d'individus par plante peut monter jusqu'à plus de 130 individus par plantes, soit plus du double que sans nourriture alternative ! Cela indique bien que ce traitement favorise l'installation d'A. swirskii sur cette culture, et améliore de fait la protection de cette culture contre les cibles de ce prédateur.

Sur une culture de concombre (FIGURE 2), en l'absence de nourriture alternative, *A*. *swirskii* n'est pas capable de se maintenir, les populations n'excèdent jamais 5 individus par plante, et ces individus proviennent de migration de plantes adjacentes la plupart du temps. En présence d'oeufs d'*A*. *ovatus* mélangés à la solution de gomme d'acacia, une installation rapide s'opère, et dans le mois suivant l'application on observe jusqu'à 20 individus par plante (avec une infestation de seulement 5 individus par plante à J0). Cette figure indique donc bien qu'en présence d'oeufs d'*A*. *ovatus* dans une composition pulvérisable mais visqueuse un traitement préventif est enfin possible avec des acariens prédateurs sur concombre sans avoir à déposer des mini-élevages (sachets) régulièrement ou à rajouter des prédateurs par un autre moyen. Les populations du prédateur, avec le traitement présenté ici, se développent et s'installent de manière durable dans la culture.

## Revendications

1. Composition aqueuse pulvérisable destinée à nourrir des agents de contrôle biologique comprenant des oeufs d'au moins une espèce d'arthropodes et un ou plusieurs épaississants et/ou gélifiants d'origine végétale choisis dans le groupe consistant en les gommes végétales, les exsudats de plantes, les galactomannanes issus de certaines graines, les glucommananes, les pectines situées dans les parois cellulaires, les extraits d'algue, les amidons naturels ou modifiés, les dérivés de la cellulose, les gels issus de production métabolique de végétaux, les maltodextrines.

2. Composition selon la revendication 1, le(les)dit(s) épaississants et/ou gélifiants étant présents en une quantité comprise entre 0,01 et 5% p/v.

3. Composition selon la revendication 1 ou 2, le(les)dit(s) épaississants et/ou gélifiants étant présents en une quantité apportant à la composition une viscosité dynamique à gradient de vitesse nulle comprise entre 0,005 et 100 Pa.s.

4. Composition selon l'une quelconque des revendications précédentes, **caractérisée en ce que** lesdits oeufs d'arthropodes appartiennent à une ou plusieurs espèce(s) d'acarien, ou une ou plusieurs espèce(s) d'insecte, ou une ou plusieurs espèce(s) de crustacé.

5. Composition selon l'une quelconque des revendications 1 à 3, **caractérisée en ce que** lesdits oeufs d'arthropodes appartiennent à au moins une espèce d'acarien et au moins une espèce d'insecte, ou au moins une espèce d'acarien et au moins une espèce de crustacé, ou au moins une espèce d'insecte et au moins une espèce de crustacé.

6. Composition selon l'une quelconque des revendications précédentes, **caractérisée en ce que** le(les)dit(s) épaississants et/ou gélifiants sont choisis dans le groupe consistant en la gomme d'acacia, la gomme de caroube, la gomme d'avoine, la gomme de guar, la gomme adragante, la gomme arabique, la gomme xanthane, la gomme karaya, la gomme tara, la gomme gellane, la gomme ghatti, la gomme de konjac, la gomme de casse.

7. Composition selon l'une quelconque des revendications 1 à 5, **caractérisée en ce que** le(les)dit(s) épaississants et/ou gélifiants sont choisis dans le groupe consistant en les maltodextrines de maïs, les maltodextrines de tapioca, les maltodextrines de pomme de terre.

8. Procédé de préparation d'une composition selon l'une quelconque des revendications 1 à 7, comprenant les étapes successives de :
- dissolution de ou des épaississants et/ou gélifiants dans une base aqueuse, de préférence dans l'eau,
- ajout des oeufs d'arthropodes à ce mélange.

9. Utilisation d'une composition selon l'une des revendications 1 à 7, en tant que source de nutriment pour des agents de contrôle biologique.

10. Utilisation d'une composition selon l'une des revendications 1 à 7, pour améliorer l'efficacité des procédés de contrôle biologique.

11. Méthode de contrôle biologique comprenant une étape d'application sur les plantes d'une composition selon l'une des revendications 1 à 7.

12. Méthode de contrôle biologique comprenant les étapes successives de :
a) application de prédateurs ou de compositions de contrôle biologique les comprenant sur des plantes;
b) application d'une composition selon l'une des revendications 1 à 7.

13. Méthode selon la revendication 11 ou 12 où l'application d'une composition selon l'une des revendications 1 à 7 est réalisée par pulvérisation.

## Patentansprüche

1. Zerstäubbare wässrige Zusammensetzung, die bestimmt ist, biologische Kontrollmittel zu nähren, umfassend Eier mindestens einer Spezies von Arthropoden und ein oder mehrere Verdickungs- und/oder Geliermittel pflanzlichen Ursprungs, ausgewählt aus der Gruppe, die aus den pflanzlichen Gummis, den Pflanzenexsudaten, den Galactomannanen bestimmter Samen, den Glucommananen, den Pektinen in den Zellwänden, den Algenextrakten, den natürlichen oder modifizierten Stärken, den Zellulosederivaten, den Gelen im Ergebnis der metabolischen Produktion der Pflanzen, den Maltodextrinen besteht.

2. Zusammensetzung nach Anspruch 1, wobei das/die Verdickungs- und/oder Geliermittel in einer Menge zwischen 0,01 und 5 % Gew./Vol. inklusive vorhanden sind.

3. Zusammensetzung nach Anspruch 1 oder 2, wobei das/die Verdickungs- und/oder Geliermittel in einer Menge vorhanden sind, die der Zusammensetzung eine dynamische Viskosität mit Geschwindigkeitsgradientem Null zwischen 0,005 und 100 Pa.s. inklusive verleihen.

4. Zusammensetzung nach einem der vorangehenden Ansprüche, **dadurch gekennzeichnet, dass** die Arthropodeneier zu einer oder mehreren Acari-Spezies oder einer oder mehreren Insekten-Spezies oder einer oder mehreren Crustacea-Spezies gehören.

5. Zusammensetzung nach einem der Ansprüche 1 bis 3, **dadurch gekennzeichnet, dass** die Arthropodeneier zu mindestens einer Acari-Spezies und mindestens einer Insekten-Spezies oder mindestens einer Acari-Spezies und mindestens einer Crustacea-Spezies oder mindestens einer Insekten-Spezies und mindestens einer Crustacea-Spezies gehören.

6. Zusammensetzung nach einem der vorangehenden Ansprüche, **dadurch gekennzeichnet, dass** das/die Verdickungs- und/oder Geliermittel aus der Gruppe ausgewählt sind, die aus dem Akazien-Gummi, dem Johannisbrotkernmehl, dem Hafer-Gummi, dem Guarkernmehl, dem Traganth, dem Gummi arabicum, dem Xanthan, dem Karayagummi, dem Tarakernmehl, dem Gellan, dem Ghatti-Gummi, dem Konjac-Gummi, dem Cassia-Gummi besteht.

7. Zusammensetzung nach einem der Ansprüche 1 bis 5, **dadurch gekennzeichnet, dass** das/die Verdickungs- und/oder Geliermittel aus der Gruppe ausgewählt sind, die aus den Mais-Maltodextrinen, den Tapioka-Maltodextrinen, den Kartoffel-Maltodextrinen besteht.

8. Herstellungsverfahren einer Zusammensetzung nach einem der Ansprüche 1 bis 7, umfassend die aufeinanderfolgenden Schritte:
- Auflösen von oder der Verdickungs- und/oder Geliermittel in einer wässrigen Basis, vorzugsweise in Wasser,
- Hinzufügen der Arthropodeneier zu diesem Gemisch.

9. Verwendung einer Zusammensetzung nach einem der Ansprüche 1 bis 7 als Nahrungsquelle für biologische Kontrollmittel.

10. Verwendung einer Zusammensetzung nach einem der Ansprüche 1 bis 7 zur Verbesserung der Wirksamkeit der biologischen Kontrollverfahren.

11. Biologische Kontrollmethode, umfassend einen Anwendungsschritt einer Zusammensetzung nach einem der Ansprüche 1 bis 7 auf den Pflanzen.

12. Biologische Kontrollmethode, umfassend die folgenden Schritte:
a) Anwendung von Prädatoren oder von biologischen Kontrollzusammensetzungen, die diese umfassen, auf Pflanzen;
b) Anwendung einer Zusammensetzung nach einem der Ansprüche 1 bis 7.

13. Methode nach Anspruch 11 oder 12, wobei die Anwendung einer Zusammensetzung nach einem der Ansprüche 1 bis 7 durch Zerstäuben durchgeführt wird.

## Claims

1. Sprayable aqueous composition intended to feed biological control agents comprising eggs of at least one arthropod species and one or more thickeners and/or gelling agents of plant origin selected from the group consisting of plant gums, plant exudates, galactomannans derived from certain seeds, glucomannans, pectins located within cell walls, algae extracts, natural or modified starches, cellulose derivatives, gels derived from metabolic production of plants, maltodextrins.

2. Composition according to claim 1, said thickener(s) and/or gelling agent(s) being present in a quantity between 0.01 and 5% w/v.

3. Composition according to claim 1 or 2, said thickener(s) and/or gelling agent(s) being present in a quantity that provides the composition with a dynamic viscosity at zero velocity gradient between 0.005 and 100 Pa.s.

4. Composition according to any one of the preceding claims, **characterized in that** said arthropod eggs are members of one or more mite species, or one or more insect species, or one or more crustacean species.

5. Composition according to any one of claims 1 to 3, **characterized in that** said arthropod eggs are members of at least one mite species and at least one insect species, or at least one mite species and at least one crustacean species, or at least one insect species and at least one crustacean species.

6. Composition according to any one of the preceding claims, **characterized in that** said thickener(s) and/or gelling agent(s) are selected from the group consisting of acacia gum, carob gum, oat gum, guar gum, gum tragacanth, gum arabic, xanthan gum, karaya gum, tara gum, gellan gum, gum ghatti, konjac gum, cassia gum.

7. Composition according to any one of claims 1 to 5, **characterized in that** said thickener(s) and/or gelling agent(s) are selected from the group consisting of corn maltodextrins, tapioca maltodextrins, potato maltodextrins.

8. Method for preparing a composition according to any one of claims 1 to 7, comprising the successive steps of:
- dissolving the thickener(s) and/or gelling agent(s) in an aqueous base, preferably in water,
- adding the arthropod eggs to this mixture.

9. Use of a composition according to one of claims 1 to 7, as a nutrient source for biological control agents.

10. Use of a composition according to one of claims 1 to 7, to improve the effectiveness of biological control methods.

11. Biological control method comprising a step of applying on the plants a composition according to one of claims 1 to 7.

12. Biological control method comprising the successive steps of:
a) applying predators or biological control compositions comprising same on plants;
b) applying a composition according to one of claims 1 to 7.

13. Method according to claim 11 or 12 where the application of a composition according to one of claims 1 to 7 is carried out by spraying.
